Europäisches Patentamt

European Patent Office

Office européen des brevets

(19)

(11) Veröffentlichungsnummer: **0 331 773**
**A1**

## (12) EUROPÄISCHE PATENTANMELDUNG

(21) Anmeldenummer: 88103625.5

(22) Anmeldetag: 08.03.88

(51) Int. Cl.4: **G01F 1/40 , G01F 1/42 , A61B 5/08**

(43) Veröffentlichungstag der Anmeldung:
13.09.89 Patentblatt 89/37

(84) Benannte Vertragsstaaten:
**AT CH DE ES FR GB IT LI NL SE**

(71) Anmelder: **PPG HELLIGE B.V.**
**I.B.C.-Weg 1**
**NL-5683 PK Best(NL)**

(72) Erfinder: **de Beer, R.H.M.**
**Domtorenstraat 18**
**NL-5037 AS Tilburg(NL)**
Erfinder: **Kuypers, M.H.**
**Schoolstraat 53**
**NL-5561 AH Riethoven(NL)**
Erfinder: **Holland, J.**
**v.d. Duyn v. Maasdamstraat 4**
**NL-4191 GC Geldermalsen(NL)**
Erfinder: **Schotte, H.**
**Goudrenetstraat 11**
**NL-5363 NT Eindhoven(NL)**

(74) Vertreter: **Patentanwälte TER MEER - MÜLLER**
**- STEINMEISTER**
**Mauerkircherstrasse 45**
**D-8000 München 80(DE)**

(54) Strömungswiderstandsrohr für Gasströmungsmesser.

(57) Das Strömungswiderstandsrohr für Gasströmungsmesser, wie sie insbesondere zur Atmungsüberwachung verwendet werden, weist ein zwischen zwei Meßanschlüssen im Bereich einer Rohrerweiterung angeordnetes Blendenelement (3) auf, das durch einen einseitig angelenkten polygonalen Ausschnitt einer quer zur Gasströmung im Rohr (1) gespannten Membran (2) gebildet ist und durch elastische Auslenkung in Strömungsrichtung einen strömungsgeschwindigkeitsabhängigen dynamischen Strömungswiderstand bildet. Zur Optimierung der Linearität eines mit einem elektronischen Differenzdruckmesser erfaßten Druckdifferenzsignals ist erfindungsgemäß die Schlitzbreite (b) der Trennungslinie (22) zwischen dem Umriß des Blendenelements (3) und der Kontur des Blendenausschnitts in der Membran (2) zwischen 0,1 bis 0,5 mm gewählt, während die Membran bei bevorzugter Verwendung einer

"Mylar®"-Folie eine Dicke zwischen 50 μm und 125 μm aufweist. Zur weiteren Verbesserung der Linearität des Druckdifferenzsignals, insbesondere bei größeren Luftdurchsätzen, sind die Übergangsbereiche an der Innenwand des Strömungswiderstandsrohrs kontinuierlich gestaltet und verrundet und für das Verhältnis des Durchmessers D der Rohrerweiterungen zur (halben) Länge L der Rohrerweiterung gilt die Beziehung $\frac{L}{D} \geq 1$.

FIG.5

## Strömungswiderstandsrohr für Gasströmungsmesser

Die Erfindung bezieht sich auf Gasströmungsmesser und betrifft insbesondere ein Strömungswiderstandsrohr als Teil eines Differenzdruckmessers für bidirektionale Gasströme nach dem Oberbegriff des Patentanspruchs 1. Ein solches Strömungswiderstandsrohr wird insbesondere in Geräten für die Messung der Atemströmung und des Atmungsdrucks benötigt.

Ein Strömungswiderstandsrohr für Gasströmungsmesser mit Differenzdruckmeßeinrichtung entsprechend dem Oberbegriff des Patentanspruchs 1 ist in der US-A-4 083 245 beschrieben. Dieser bekannte Differenzdruckmesser weist ein in einen Gasströmungskanal eingesetztes Strömungswiderstandselement in Form eines Rohrs auf, das auch als "passiver Wandler" bezeichnet werden könnte. In diesem Strömungswiderstandsrohr ist gleichabständig zwischen zwei Meßanschlüssen ein elastisches Blendenelement angeordnet, das durch einen einseitig angelenkten polygonalen Ausschnitt einer quer zur Gasströmung gespannten Membran aus einem bestimmten Kunststoffmaterial gebildet ist und das einen elastisch rückstellenden dynamischen Strömungswiderstand darstellt, der sich durch unterschiedlich starke Auslenkung des Blendenelements in Strömungsrichtung weitgehend strömungsgeschwindigkeitslinear ändert. Der polygonale Schnitt des Blendenelements kann beispielsweise die Form eines Rhombus aufweisen, der entlang einer Schmalseite mit dem übrigen Teil der Membran verbunden ist; es wird insoweit insbesondere auf die Fig. 2 der genannten Druckschrift US-A-4 083 245 verwiesen. Gegenüber den aus US-A-2 989 866 und US-A-3 989 037 bekannten elastisch verformbaren Strömungswiderständen solcher passiver Wandler besitzt ein nach der US-A-4 083 245 aufgebauter Gasströmungsmesser den Vorteil, daß das erzielbare Druckdifferenzsignal in Abhängigkeit von der Gasströmungsgeschwindigkeit bzw. dem Gasdurchsatz linearer ist.

Der Erfindung liegt die Aufgabe zugrunde, einen Differenzdruckmesser der beschriebenen Art aus verschiedenen nachfolgend erläuterten Gründen zu verbessern.

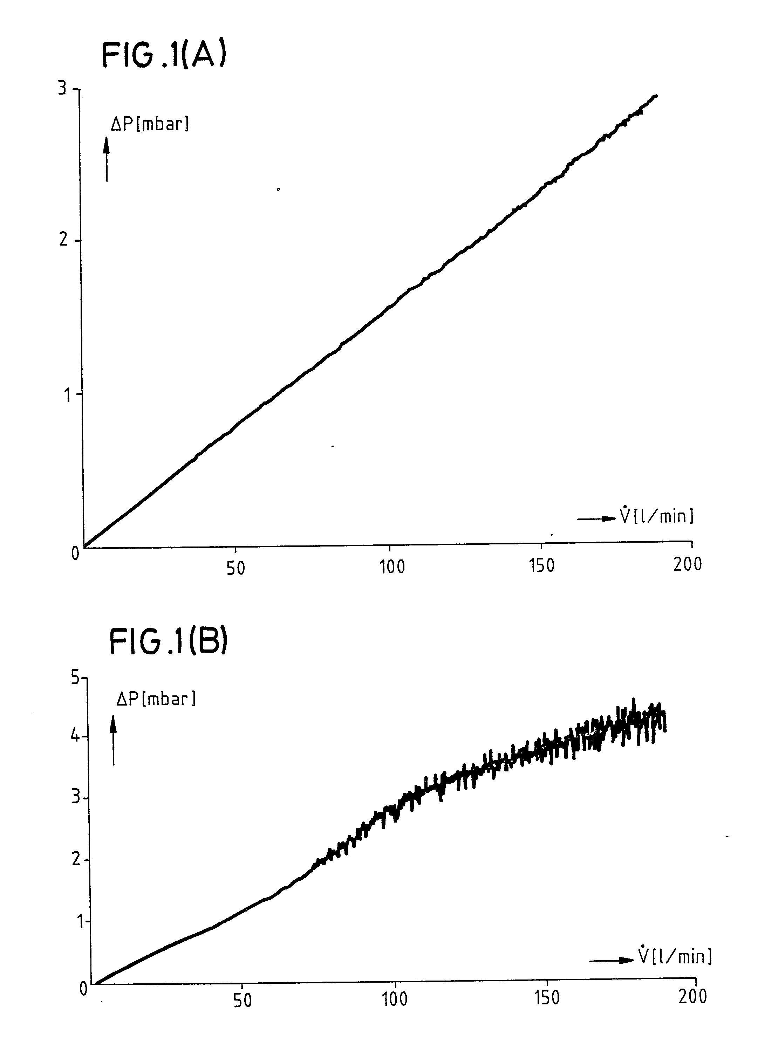

So hat sich, wie sich aus dem Diagramm der beigefügten Fig. 1(B) ersehen läßt, gezeigt, daß sich mit dem bekannten Strömungsmesser im Bereich kleinerer Luftdurchsätze bis etwa 80 l/min eine relativ gut lineare Abhängigkeit des Druckverlusts bzw. Differenzdrucks erreichen läßt. Bei größeren Gas-, insbesondere Luftdurchsätzen jedoch ist einerseits der Differenzdruck nicht mehr linear zur Strömungsgeschwindigkeit und zum anderen treten Instabilitäten auf.

Weiterhin ist das Differenzdrucksignal bei sehr kleinen Luftdurchsätzen im Bereich unter 10 l/min deutlich nichtlinear, wenn der Strömungswiderstand entsprechend der Lehre der US-A-4 083 245 hergestellt wird.

Für die Nichtlinearität des Druckdifferenzsignals bei größeren Luftdurchsätzen, insbesondere für die zunehmende rasche Fluktuation, die sich gut aus Fig. 1(B) ersehen läßt, war zunächst keine eindeutige Erklärung zu ermitteln, zumal diese Probleme in der genannten US-Patentschrift nicht angesprochen sind. Als eine Ursache ließ sich die bisher übliche Verwendung von längeren flexibel nachgiebigen Kunststoffschläuchen ermitteln, die zwischen dem Strömungswiderstandsrohr und dem eigentlichen Differenzdruckmeßgerät vorgesehen sind. Eine Lösung für dieses Problem wird in einer zeitlich gleichrangigen europäischen Patentanmeldung der gleichen Anmelderin angeboten. Als weitere Ursache ließen sich Turbulenzen im Strömungswiderstandsrohr ausmachen. Das bekannte Strömungswiderstandsrohr weist zwischen einem Einlaß- und Auslaßbereich eine Rohrerweiterung auf, in deren axialer Mittenposition die quer zur Strömung gespannte Membran mit Blendenelement angeordnet ist. Im Übergangsbereich zwischen dem engeren Ein- bzw. Auslaß und dem erweiterten Bereich des Rohrs kann es bei dem bekannten Strömungswiderstandsrohr zu Wirbelbildungen kommen. Solche Wirbel sind unter anderem Ursache für die Fluktuation des Druckdifferenzsignals bei größeren Gasdurchsätzen.

Im Bereich sehr kleiner Luftdurchsätze, insbesondere unter 10 l/min, ließ sich aber eine Begründung für die Nichtlinearität des Druckdifferenzsignals in Abhängigkeit vom Luftdurchsatz überhaupt nicht erkennen. In der genannten US-Patentschrift ist vielmehr ausgeführt, daß sich bei Verwendung von hochelastischen Kautschukmembranen mit einer Dicke von 0,9 mm und bei einer Schlitzbreite von 0,7 mm eine befriedigende Linearität des Druckdifferenzsignals erreichen lasse, wobei unter Schlitzbreite der Abstand zwischen der Umrißlinie des Blendenelements und der umgebenden Kontur der restlichen Membran verstanden wird. Genauere Untersuchungen und Versuche haben jedoch ergeben, daß sich bei Einhaltung dieser Parameter auch bei Verwendung der verschiedensten Membranmaterialien, Variation des Membrandurchmessers sowie unterschiedliche Größe und Kontur des Blendenelements keine befriedigende Linearität des Druckdifferenzsignals in Abhängigkeit vom Luftdurchsatz bei kleinen Durchsatzvolumina erreichen ließ.

Aus diesen Erkenntnissen folgte die der Erfindung zugrundeliegende Lösungsidee, eine der Lehre des Patentanspruchs 1 entsprechende konstruktive Gestaltung der Membran und des Blendenelements zu verwenden.

Mit der Erfindung und den in abhängigen Patentansprüchen angegebenen Ergänzungen und Verbesserungen des Erfindungsgedankens wird der angestrebte technische Erfolg und Vorteil erreicht, daß einmal der Frequenzgang bei strenger Linearität des Meßsignals bei größeren Luftdurchsätzen wesentlich verbessert wird, wobei durch Verlängerung des Gehäuses in Relation zu dessen Durchmesser sowie durch Verrunden aller Übergangsquerschnitte an der Innenwand des Gasströmungsrohrs des Strömungswiderstandselements zusätzliche Wirbelbildung vermieden wurde, so daß besondere Glättungsmaßnahmen für das Druckdifferenzsignal nicht mehr erforderlich sind. Bei sehr kleinen Luftdurchsätzen dagegen wurde durch eine der Lehre des Patentanspruchs 1 entsprechende Wahl der Dicke der Membran und der Schlitzbreite zwischen dem Blendenelement und der Membran eine deutliche Linearitätsverbesserung erzielt.

Die Erfindung und vorteilhafte Einzelheiten werden nachfolgend unter Bezug auf die Zeichnung in beispielsweiser Ausführungsform erläutert. Es zeigen:

Fig. 1 zwei vergleichende Diagramme für das Druckdifferenzsignal in Abhängigkeit vom Luftdurchsatz beim besten derzeit bekannten Stand der Technik (Fig. 1(B)) bzw. bei der Erfindung (Fig. 1(A));

Fig. 2 und 3 je ein Diagramm zur Erläuterung bestimmter Vorteile der Erfindung;

Fig. 4 konstruktive Einzelheiten zur vorteilhaften Gestaltung eines Strömungswiderstandsrohrs gemäß der Erfindung; und

Fig. 5 ein Ausführungsbeispiel für eine vorteilhafte Gestaltung eines in der Ebene der Membran als Strömungswiderstand liegenden Blendenelements.

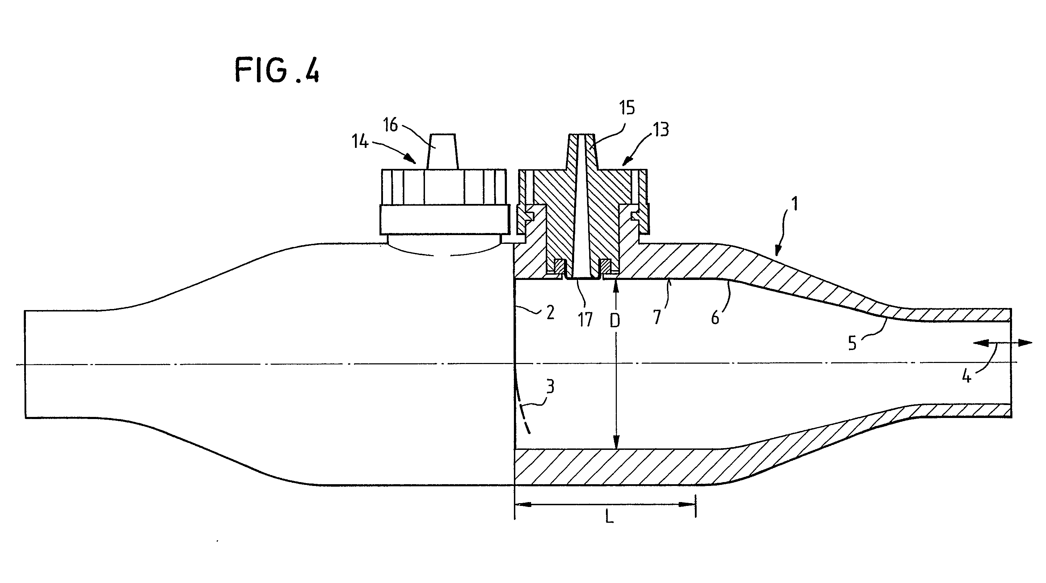

Die Vergleichsdiagramme (A) und (B) der Fig. 1 wurden bereits oben erläutert. Die Fig. 1(A) läßt deutlich erkennen, wie als Folge der erfindungsgemäßen Auswahl der Membrandikke, der Schlitzbreite zwischen der Umrißlinie des Blendenelements und dem übrigen Teil der Membran und schließlich durch Aufweitung und Verlängerung des Gehäuses sowie Verrunden der Übergangsbereiche 5 und 6 in Fig. 4 zwischen dem Einlaß 4 bzw. Auslaß und einer Rohrerweiterung 7 einerseits ein fast lineares Druckdifferenzsignal in Abhängigkeit von der Gasströmungsgeschwindigkeit erreicht wird und andererseits bei höheren Luftdurchsätzen höherfrequente Fluktuationen des Meßsignals nicht mehr auftreten. Gute Signallinearität wird insbesondere erzielt, wenn für das Verhältnis der (halben) Gehäuselänge L zum lichten Durchmesser D der Rohrerweiterung (siehe Fig. 4) die Beziehung gilt: $\frac{L}{D} \geq 1$.

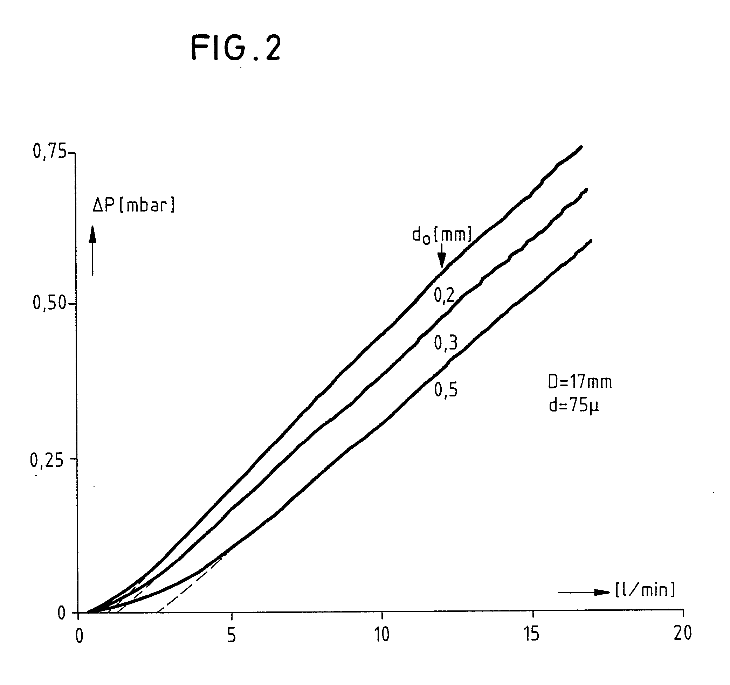

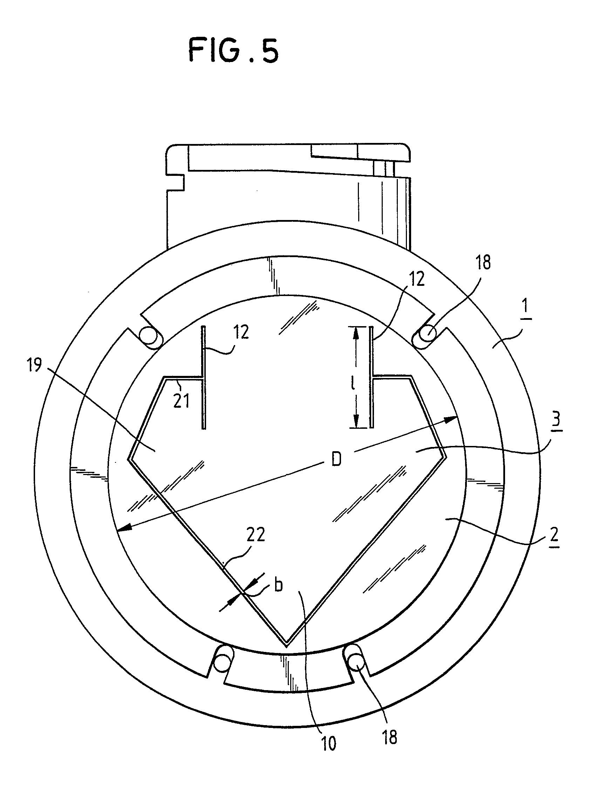

Die Fig. 2 verdeutlicht bei einem für die Pädiatrie bestimmten Gasströmungsmesser bei einem Durchmesser des Blendenelements 2 (Fig. 4 und 5) von D = 17 mm und einer als optimal ermittelten Membrandicke von d = 75 $\mu$m den Zusammenhang des Druckdifferenzsignals $\Delta P$ in Abhängigkeit von kleinen Luftdurchsatzvolumina, wobei die Schlitzbreite $d_0$ als Parameter aufgetragen ist. Es läßt sich deutlich erkennen, daß bei zunehmender Schlitzbreite eine noch stärkere Nichtlinearität bzw. ein nur langsam ansteigendes Druckdifferenzsignal in Abhängigkeit vom Luftdurchsatz zu erwarten ist. Eine fast vollständige Linearität bis in den Bereich sehr kleiner Luftdurchsätze ließ sich bei einer Schlitzbreite von ca. 0,2 mm erreichen, was aus dem Diagramm der Fig. 2 gut ersichtlich ist, das bei Verwendung einer Membran und Blende aus dem unter dem Handelsnamen "Mylar" bekannten Kunststoffmaterial gemessen wurde.

Bei noch kleineren Schlitzbreiten entsteht eine Art Haftungseffekt, d. h. zur Auslenkung des Blendenelements 3 (Fig. 4 und 5) wird auch bei sehr sauberer Schnittkontur eine bestimmte Schwelle der Luftströmung benötigt, um ein nachweisbares Druckdifferenzsignal messen zu können.

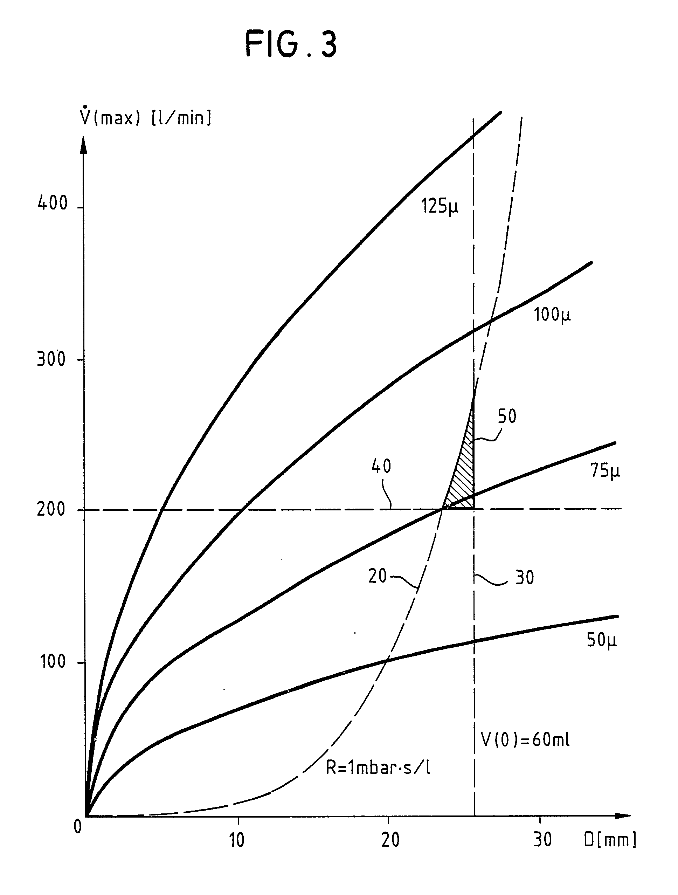

Aus den Kurvenscharen der Fig. 3 läßt sich ein optimaler Bereich für die hier primär vorgesehene medizinische Anwendung eines erfindungsgemäßen Strömungswiderstandsrohrs im Bereich der Atmungsüberwachung, und zwar sowohl für die Pädiatrie als auch für die Messung der Atemströmung bzw. des Atmungsdrucks bei Erwachsenen ableiten. Den in Fig. 3 dick ausgezogenen Kurvenscharen liegt als Parameter die Dicke d der Membran (wiederum aus "Mylar") zugrunde, nämlich in einem Bereich von 50 $\mu$m $\leq$ d $\leq$ 125 $\mu$m. Eine gestrichelt eingezeichnete Exponentialkurve 20 kennzeichnet die Grenzlinie für den zulässigen maximalen Atemwiderstand bei der Ein-und Ausatmung, der im dargestellten Beispiel mit R = 1 mbar . sec/l angegeben ist. Ein maximal zulässiges Totvolumen für das Gesamtsystem aus Strömungswiderstandsrohr, Anschlüssen, Leitungsverbindungen und Druckkammern im elektronischen Drucksensor ist - veranschaulicht durch eine vertikal verlaufende Grenzlinie 30 - mit V(0) = 60 ml angegeben, wobei der Wert von 60 ml etwa dem Lungeninhalt eines Neugeborenen entspricht. Schließlich markiert eine bei einer Maximalströmungsgeschwindigkeit V = 200 l/min angegebene horizontale Grenzlinie 40 den Wert der geforderten Untergrenze für eine sinnvolle Messung im Bereich der Atmungsüberwachung.

Der optimale medizinische Bereich für einen Differenzdruckmesser für bidirektionale Gasströme mit einem Strömungswiderstandsrohr erfindungsgemäßer Art ist damit durch den in Fig. 3 schraffierten Bereich 50 angegeben, der durch die Kurve 20 sowie die beiden Geraden 30 und 40 begrenzt ist. Dieser optimale Bereich liegt ersichtlicherweise zwischen Dickenwerten für die Membran von 70 $\mu m \leq d \leq 90 \mu m$, und zwar innerhalb relativ weiter Grenzen für den Durchmesser D der Membran. Es wurden Untersuchungen für Durchmesserbereiche der Membran im Bereich von D = 10 mm bis D = 30 mm durchgeführt. Für eine Pädiatrieversion eines Gasströmungsmessers erwies sich ein Durchmesser der Membran von D = 17 mm als besonders vorteilhaft, während für die normale klinische Version eines solchen Gasströmungsmessers ein Membrandurchmesser von D = 24 mm als vorteilhaft ermittelt wurde.

Die Kurvenscharendarstellung der Fig. 3 gilt für Materialien der Membran 2 bzw. des Blendenelements 3 mit einem Elastizitätsmodul, der in etwa dem Elastizitätsmodul von "Mylar" (Handelsname) entspricht. Das Material "Mylar" hat für den hier vorliegenden Anwendungszweck den Vorteil, daß die Abhängigkeit der Elastizität (des Elastizitätsmoduls) von der (relativen) Beanspruchung über vergleichsweise sehr lange Zeiträume linear ist.

Die Kurvenscharen der Fig. 3 lassen die Grenzbedingungen für Folienmaterialien der Membran bzw. des Blendenelements erkennen, deren Eigenschaften jenen von "Mylar" entsprechen. Das Diagramm veranschaulicht den maximal meßbaren Fluß als Funktion des Durchmessers D (vgl. Fig. 4 und 5) mit der Dicke des Blendenelements bzw. der Membran als Parameter ($\dot{V}_{(max)} = f(D)_d$).

Die Kurvenschardarstellung ist bezogen auf einen (relativen) maximal zulässigen Linearitätsfehler von $f \leq 3$ %.

Die vergrößerte Schnittdarstellung der Fig. 4 eines Strömungswiderstandsrohrs mit erfindungsgemäßen Merkmalen zeigt deutlich die kontinuierlichen verrundeten Übergänge 5 und 6 im Bereich des Übergangs von einem Ein- bzw. Auslaßbereich 4 zu dem erweiterten Bereich 7, der mittig durch die eingespannte Membran 2 unterteilt ist, aus der das Blendenelement 3 herausgeschnitten ist. Die Meßanschlüsse in Gasströmungsrichtung vor bzw. hinter dem Blendenelement 3 sind in Fig. 4 mit den Bezugshinweisen 13 bzw. 14 gekennzeichnet. Die Verbindung zwischen dem Strömungswiderstandsrohr 1 und einem elektronischen Differenzdrucksensor (nicht gezeigt) ist als Konus 15 bzw. 16 verwirklicht, in dessen dem Inneren des Strömungswiderstandsrohrs 1 zugewandten Ende je ein hydrophobes Filter 17 eingesetzt ist, das gleichzeitig eine Bakteriensperre darstellt zur Trennung des relativ schlecht zu reinigenden Meßkreises des Differenzdrucksensors vom Patientenkreis, in dem das Strömungswiderstandsrohr 1 liegt.

Die Fig. 5 zeigt in schematischer Darstellung ein vorteilhaftes Ausführungsbeispiel für den Umriß des in der Ebene der Membran 2 liegenden Blendenelements 3 in der axialen Mitte zwischen den beiden Hälften des Gehäuses des Strömungswiderstandsrohrs 1. Die Membran 2 ist zwischen den beiden Gehäusehälften durch Zapfen oder Ansätze 18 locker gehalten und dann unter Klemmwirkung der beiden Gehäusehälften so eingespannt, daß sich keine Zugspannung in irgendeiner radialen Richtung ergibt.

Der Schnitt der Blende 3 läßt eine fünfeckige Konfiguration erkennen mit einem größeren, nach unten spitzwinklig zulau fenden unteren Blendenteil 10 und einem oberen breiteren Blendenteil 19. Entlang der oberen Querkante 21 ist das Blendenelement 3 über den mittleren Teil seiner Länge integral mit der Membran 2 verbunden. Durch zwei in Vertikalrichtung stehende Einschnitte 12 wird einerseits eine hohe Flexibilität des Blendenelements 3 erreicht und außerdem läßt sich über die Einschnittiefe eine im Hinblick auf die Linearität des Druckdifferenzsignals günstige Federkonstante einstellen, wobei, wie bereits erwähnt, eine spannungsfreie Einklemmung der Membran 2 vorausgesetzt wird.

Die optimale Länge der Einschnitte 12 ist etwas vom Durchmesser des Blendenelements 2 abhängig. Im Hinblick auf eine Optimierung der Linearität des Druckdifferenzsignals wurden durch Versuche Verhältniswerte von $0,15 \leq \ell.D \leq 0,4$ ermittelt, wobei mit $\ell$ die Länge der beiden Einschnitte 12 und mit D der Durchmesser des Blendenelements 2 bezeichnet sind. Wie die Zeichnung erkennen läßt, liegen die Einschnitte 12 vorzugsweise symmetrisch zu einer gedachten Vertikallinie durch die untere Spitze des Blendenelements 3 und jeweils etwa zur Hälfte ober- bzw. unterhalb der oberen Querkante 21 der Umrißlinie des Blendenelements 3.

Die Breite des Schlitzes 22, d. h. des Abstands zwischen der Umrißlinie des Blendenelements 3 und der gegenüberliegenden umlaufenden Kante des restlichen Teils der Membran 2 liegt, wie im Patentanspruch angegeben und oben erläutert, vorzugsweise im Bereich von $0,1$ mm $\leq b \leq 0,5$ mm. Sehr günstige Verhältnisse hinsichtlich der Linearität des Druckdifferenzsignals insbesondere bei kleinen Luftdurchsätzen ergeben sich für einen Wert der Schlitzbreite im Bereich $0,15 \leq b \leq 0,3$, insbesondere für den optimalen Wert von b = 0,2 mm.

Für die optimalen (relativen) Abmessungen des aufgeweiteten Bereichs 7 zu dessen Länge gilt L.D $\geq 1$, wie bereits er wähnt.

Eine zusätzliche Optimierung der Linearität des Druckdifferenzsignals läßt sich erreichen, wenn die Schlitzbreite b über die gesamte Länge der Umrißlinie des Schlitzes 22 variiert mit einer etwas geringeren Schlitzbreite im Bereich der integralen Verbindung mit der Membran 2 und einer etwas größeren Schlitzbreite im nach unten spitzwinklig zulaufenden unteren Blendenteil 10.

Zusammenfassend läßt sich feststellen, daß mit der Erfindung eine deutliche Verbesserung hinsichtlich des Frequenzgangs und der Linearität des Druckdifferenzsignals durch eine verbesserte konstruktive Gestaltung des Strömungswiderstandsrohr erreicht wird im Vergleich zu einem Strömungswiderstandsrohr, wie es in der US-A-4 083 245 be-schrieben ist.

## Ansprüche

1. Strömungswiderstandsrohr für Gasströ-mungsmesser mit einem zwischen zwei Meßanschlüssen im Bereich einer Rohrerweiterung (7) angeordneten Blendenelement (3), das durch einen einseitig angelenkten polygonalen Ausschnitt einer quer zur Gasströmung im Rohr (1) gespannten Membran (2) gebildet ist und durch elastische Aus-lenkung einen strömungsgeschwindigkeitsabhängi-gen (dynamischen) Strömungswiderstand bildet, **dadurch gekennzeichnet, daß**
- die Schlitzbreite (b) der Trennungslinie (22) zwischen dem Umriß des Blendenelements (3) und der Kontur des Blendenausschnitts in der Membran (2) zwischen 0,1 und 0,5 mm liegt, und daß
- die Membran (2) zwischen 50 μm und 125 μm dick ist.

2. Strömungswiderstandsrohr nach Anspruch 1, **dadurch gekennzeichnet,** daß die Membran (2) mit Blendenelement (3) aus einem langzeit-hoch-elastischen Kunststoffolienmaterial besteht, insbe-sondere aus Mylar.

3. Strömungswiderstandsrohr nach Anspruch 1 oder 2, **dadurch gekennzeichnet,** daß die Innenwandung des Rohrs (1) in allen Übergangsbereichen (5, 6) zwischen Ein- bzw. Auslaß (4) und der Rohrerweiterung (7) glatt und mit verrundeten Übergängen gestaltet ist und daß für das Verhältnis zwischen dem Durchmesser D der Rohrerweiterung (7) und der Länge L der Rohrerweiterung vom Ein-bzw. Auslaß bis zur Membran (2) die Beziehung gilt: $\frac{L}{D} \geq 1$.

4. Strömungswiderstandsrohr nach Anspruch **1,** **dadurch gekennzeichnet,** daß die Schlitzbreite (b) ca. 0,2 mm beträgt.

5. Strömungswiderstandsrohr nach Anspruch 2, **dadurch gekennzeichnet,** daß die Membran (2) 75 μm bis 100 μm dick ist.

6. Strömungswiderstandsrohr nach Anspruch 1, **dadurch gekennzeichnet,** daß die Schlitzbreite (b) entlang der Umrißlinie des Blendenelements (3) unterschiedlich ist.

7. Strömungswiderstandsrohr nach Anspruch 6, **dadurch gekennzeichnet,** daß die Schlitzbreite (b) in Bereichen des Schlitzes (22) nahe der Anlenkung symmetrisch gleich, jedoch geringer ist als im übrigen Teil der Schlitzlinie.

8. Strömungswiderstandsrohr nach einem der vorstehenden Ansprüche, **gekennzeichnet durch** je einen quer zum Schlitzende verlaufenden, an gegenüberliegenden Stellen der Anlenkung des Blendenelements (3) angeordneten Einschnitt (12).

9. Strömungswiderstandsrohr nach Anspruch 8, **dadurch gekennzeichnet,** daß die Länge (1) der Einschnitte im Verhältnis zum Durchmesser (D) der Membran in einem Bereich von $0,15 \leq l/D \leq 0,4$ liegt.

10. Strömungswiderstandsrohr nach Anspruch 9, **dadurch gekennzeichnet,** daß das Verhältnis $l/D$ etwa 0,3 beträgt.

## FIG .1(A)

## FIG .1(B)

FIG.2

FIG.3

FIG.4

# FIG.5

<table>
<thead>
<tr><th colspan="3" style="text-align:center">Europäisches Patentamt<br>EUROPÄISCHER RECHERCHENBERICHT</th><th>Nummer der Anmeldung<br>EP 88 10 3625</th></tr>
</thead>
</table>

# EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | FR-A-2 360 063 (RESEARCH DEVELOPMENT CORP.)<br>* Insgesamt *<br>--- | 1,2 | G 01 F 1/40<br>G 01 F 1/42<br>A 61 B 5/08 |
| D,A | US-A-3 989 037 (FRANETZKI)<br>* Spalte 2, Zeilen 29-44; Figuren *<br>----- | | |

**RECHERCHIERTE SACHGEBIETE (Int. Cl.4)**

G 01 F
A 61 B

Der vorliegende Recherchenbericht wurde für alle Patentansprüche erstellt

| Recherchenort | Abschlußdatum der Recherche | Prüfer |
|---|---|---|
| DEN HAAG | 14-11-1988 | ROSE A.R.P. |